# EUROPEAN PATENT SPECIFICATION

(11) **EP 2 063 835 B1**
(45) Date of publication and mention of the grant of the patent: **16.11.2011**
(21) Application number: 08702413.9
(22) Date of filing: 14.01.2008
(51) Int. Cl.: A61F 13/15

(54) **DISPOSABLE ABSORBENT PRODUCT HAVING A GRAPHIC INDICATOR**
SAUGFÄHIGER EINWEGARTIKEL MIT GRAFISCHEM INDIKATOR
PRODUIT ABSORBANT JETABLE AVEC INDICATEUR GRAPHIQUE

(30) Priority: 06.03.2007 US 905341 P; 30.04.2007 US 799381
(43) Date of publication of application: 03.06.2009
(73) Proprietor: KIMBERLY-CLARK WORLDWIDE, INC., Neenah, WI 54956 (US)
(72) Inventor: WILKES, Todd, Appleton, Wisconsin 54914 (US); MLINAR, Joseph, Appleton, Wisconsin 54915 (US); ROOYAKKERS, Jon, P., Appleton, Wisconsin 54915 (US)
(74) Representative: Mabey, Katherine Frances
(86) International application number: PCT/IB2008/050113
(87) International publication number: WO 2008/107804

(56) References cited:
- EP-A- 0 756 855
- EP-A- 1 147 755
- EP-A- 1 642 557
- EP-A- 1 839 634
- WO-A-2007/024327
- US-A- 4 810 574
- US-A- 5 531 731
- US-A1- 2004 102 748
- US-A1- 2005 170 726

## Description

People use health, hygiene, and wellness products, such as disposable absorbent articles, to help live their every day lives. Examples of disposable absorbent products include feminine-care articles such as pads, infant-care articles such as diapers and training pants, specialized articles such as disposable absorbent pants for children experiencing bed-wetting problems, and adult-care articles such as incontinence articles.

Often such articles have a front portion that is designed to face the front of the body of a user when the article is worn and, correspondingly, a back portion that is designed to face the back of the body of a user when the article is worn. For those articles that are worn like underwear, the front portion is attached to the back portion in a way that defines a waist opening and two leg openings in the article as a whole. For other articles, such as a feminine or incontinence pad that is attached to a woven undergarment, the article may be shaped such that it has a front portion that is designed to face some portion of the front of the body of a user, and a back portion that is designed to face some portion of the back of the body of a user.

EP 0756855A1 discloses a disposable diaper having fastening means. US 2005/0170726A1 discloses multilayer nonwoven products including color schemes. WO2007/024327 discloses an absorbent article formed from a colored, elastomeric nonwoven material. EP 1147755A2 discloses an absorbent article having a backsheet printed with a multicolor pattern. EP 1642557A1 discloses a process of producing a breathable sheet. US 2004/102748 discloses a package of disposable absorbent articles.

At times it may be difficult for a user of the article to readily distinguish between the front portion and back portion of a disposable absorbent article. Accordingly, some disposable absorbent articles have a mark to help a user distinguish between the front portion of an article and a back portion of an article. Typically such marks have been printed onto some part of the disposable absorbent article.

The present invention provides a disposable absorbent article in accordance with claim 1, and a method of forming and packaging a disposable absorbent article in accordance with claim 5.

We have found that equipment, such as a slot coater, can be used to dispose a colored hot-melt adhesive on the waistband portion of a disposable absorbent article so as to form a graphic that helps a user to distinguish between the front portion and back portion of a disposable absorbent article. Generally the equipment and materials used to impart a graphic comprising a colored hot-melt adhesive should be cheaper than the equipment and materials used to print a graphic. Furthermore, a graphic comprising a colored hot-melt adhesive can serve the purpose of providing information to a user (in this case by helping a user distinguish between the front portion and back portion of a disposable absorbent article).

Alternatively a continuous web (e.g., a colored nonwoven web or film) may be cut (e.g., by a rotary cutting device or other such cutting or slitting equipment) to form shapes, such as squares or rectangles, that are attached to the waistband portion of the disposable absorbent article. For example, a rectangular piece of a colored film or nonwoven web may be inserted between, and attached to, nonwoven webs that form the waistband portion of a disposable absorbent article. The colored film or nonwoven web-a graphic-is visible through the nonwoven web that forms the exterior of the waistband (that is the side opposite the body-facing side of the waistband), thereby helping users distinguish between the front portion and back portion of the disposable absorbent article.

One version of the invention is a disposable absorbent article having a front portion, a back portion attached to the front portion in a way that defines a waist opening and two leg openings in the disposable absorbent article as a whole, and a graphic comprising a colored hot-melt adhesive. The shape and location of the graphic is chosen to convey information, or a signal, to a user of the disposable absorbent article so that the user can distinguish between the front portion and back portion of the article. For example, a colored hot-melt adhesive in the shape of a rectangle, circle, triangle, square, line, stripe, or other shape may be attached to the back portion of the disposable absorbent article. The user may then be instructed, e.g., by instructions given on a package containing the article, that the graphic signifies the back portion of the disposable absorbent article. The user of the article would then know that when donning the article, that portion of the disposable absorbent article bearing the identified graphic is to be oriented so as to face the back of the body of the user.

Note that the graphic need not be placed on the back portion of the article. Instead a graphic comprising a colored hot-melt adhesive can be attached to the front portion of the article, with corresponding instructions that the graphic signifies to a user that which is the front portion of the article.

The invention provides a disposable absorbent article having an attached or integral waistband, with the graphic disposed on said waistband. For example, a colored hot-melt adhesive can be placed in the middle of the waistband in the back portion of a disposable absorbent article, with the graphic signifying that the portion of the article to which it is attached is the back portion of the article.

Because of the nature of materials commonly used when constructing disposable absorbent articles, for example relatively thin nonwoven materials, the colored hot-melt adhesive need not be disposed on an outer surface of the disposable absorbent article to be seen (e.g., on the body-facing surface, or on the opposing surface that will likely face and contact any garment worn by a user of the disposable absorbent article over said article). For example, in some embodiments of disposable absorbent articles that are worn like underwear (i.e., the disposable absorbent article has a waist opening and two leg openings), two nonwoven facings are adhesively attached to one another in the waistband portion of the disposable absorbent article. One or more elastic strands or ribbons are sandwiched between these facings to impart elastic properties to the resulting laminate. I.e., the strand is attached to, and sandwiched between, the two nonwoven webs while the strand is in a stretched condition. The resulting laminate is then allowed to retract, thereby producing a gathered, elastic composite. In embodiments, a colored hot-melt adhesive is slot coated or otherwise disposed on an inner surface of one of the aforementioned webs before the webs are adhesively joined to one another. In this instance, the colored hot-melt adhesive may help join the webs together (and also possibly to one or more elastic strands or ribbons) and, because the nonwoven webs are sufficiently thin, results in a graphic that is visible to a user of the article. As noted above, the colored hot-melt adhesive can be applied in the shape of a square, rectangle, circle, triangle, line, stripe, or other shape.

The present invention also covers methods for making a disposable absorbent article comprising a graphic composed of a colored hot-melt adhesive (or colored nonwoven web or film), wherein the graphic is adapted to convey a signal to a user of the article that helps the user distinguish between the front portion and back portion of the article.

In one exemplary method, a slot coater is used to dispose a colored hot-mclt adhesive on the surface of a web that is subsequently used in the assembling of the disposable absorbent article. The colored hot-mclt adhesive can be applied to form different shapes, such as those given above. The resulting disposable absorbent article has a front portion and a back portion, with the graphic disposed on a portion thereof. In subsequent converting steps, the article is placed in a package. On the package are one or more statements instructing a user that the graphic enables the user to distinguish between the back portion and front portion of the disposable absorbent article. Or an appropriate unit operation, e.g. a cut-and-place module or analogous equipment, may be used to attach discrete lengths of a colored nonwoven web or film as the graphic that enables a user to distinguish between the back portion and front portion of the disposable absorbent article.

The present invention also covers exemplary methods for facilitating the donning of a disposable absorbent article having a front portion and a back portion. In one exemplary method, a disposable absorbent article having a front portion, a back portion, and a graphic comprising a colored hot-melt adhesive (or colored film or nonwoven web), with the graphic being adapted to convey a signal enabling a user to distinguish between the back portion and front portion of the disposable absorbent article, is placed in the hands of a user. The user is then instructed, e.g. by statements on a package containing the disposable absorbent article, to don said article with the graphic oriented so that the front portion faces the front of the body of the user and the back portion faces the back of the body of the user. For example, if the graphic is disposed on the back portion of the article, the user would be instructed to don the article with the graphic behind the person (or with the graphic oriented so that the graphic was closer to the back of the body of the person).

Applicable versions of the aforementioned embodiments may also encompass employing a plurality of slot-coating unit operations and/or a plurality of graphics. For example, one slot-coater may be used to dispose a first graphic on a first web, and a second slot-coater may be used to dispose a second graphic on a second web. The second web may be the same or different from the first web. The first and second graphic may be the same or different relative to color, shape, etc. In one version of the invention, a colored hot-melt adhesive might be intermittently applied to dispose a shape serving as a graphic indicator (e.g., a rectangle) onto a substrate or web, with this graphic indicator serving as the first graphic. Another colored hot-melt adhesive might be continuously applied to dispose one or more continuous stripes on a substrate or web, with the stripe or stripes serving as a second graphic. This web or webs might then be assembled so that, in the finished article, the shape serving as a graphic indicator is disposed in the waist region in the back portion of the article. And the stripe or stripes, or second graphic, is disposed proximate to, and substantially around the circumference of, the waist. Of course many color or non-color combinations, shape configurations, and article locations (i.e., where the graphic is disposed on the article in finished form) may be employed when forming a first and second graphic. It should be noted too that a first and second graphic might also be formed using a single slot-coating unit operation (e.g., different colored hot-melt adhesives may be directed to different locations of a mouthpiece-i.e., the openings through which the colored hot-melt adhesive or adhesives pass when disposed or applied to a web or substrate).

### FIGURES

Figure 1A depicts one representative version of a disposable absorbent article of the present invention.
Figure 1B depicts one representative version of a disposable absorbent article of the present invention.
Figure 1C depicts one representative version of a disposable absorbent article of the present invention.
Figure 1D depicts one representative version of a disposable absorbent article of the present invention.
Figure 2 depicts one representative version of a method for disposing a colored, hot-melt adhesive onto the surface of a moving web.
Figure 3 depicts one representative version of a method for disposing a colored, hot-melt adhesive onto the surface of a moving web.
Figures 4A, 4B, and 4C depict representative versions of openings / mouthpieces through which a colored-hot melt adhesive may be directed when being disposed on the surface of a substrate.

### DESCRIPTION

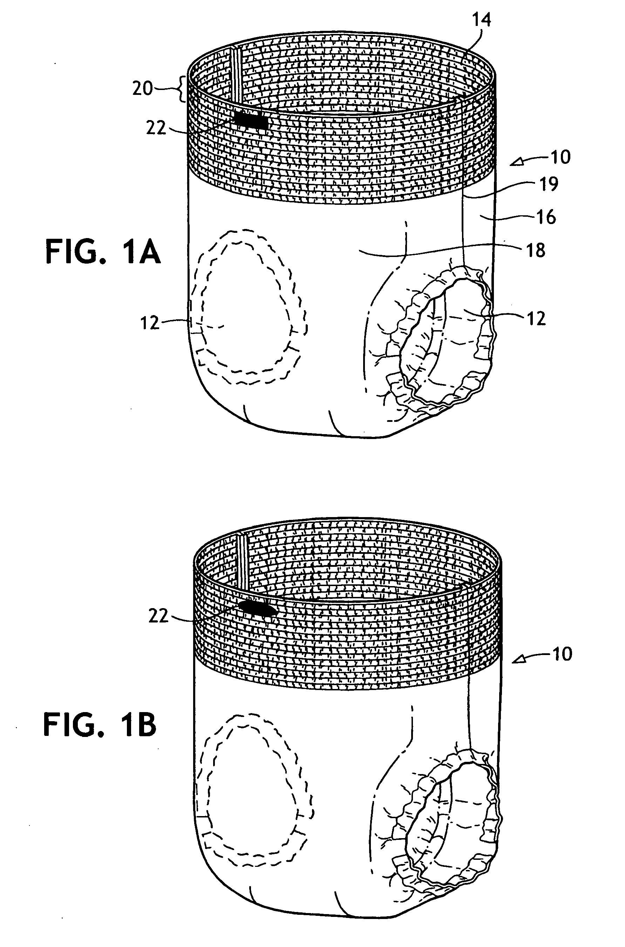

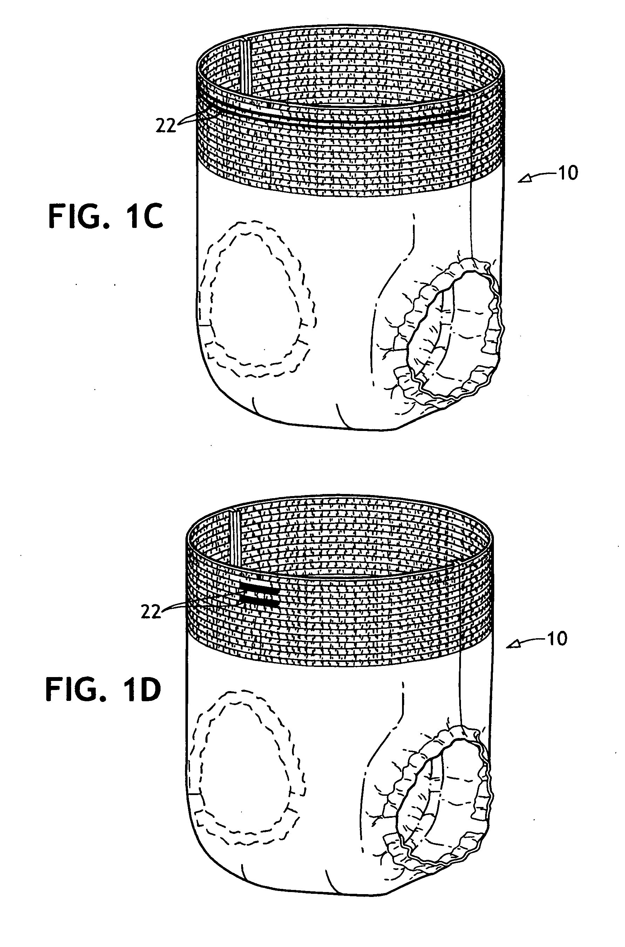

Figures 1A, 1B, 1C, and 1D each depict one representative version of a disposable absorbent article of the present invention. In Figure 1A, a disposable absorbent article 10 is depicted, with the article defining two leg openings 12 and a waist opening 14. The disposable absorbent article has both a front portion 16 and a back portion 18. The front portion and back portion are joined at seam 19 (e.g., by using heat; other forms of energy, such as ultrasonics; adhesive; or other such materials/techniques for joining materials). The front portion and back portion of the disposable absorbent article need not be attached directly to one another. These portions may be attached indirectly to one another. The depicted article also comprises an integral waistband 20. In the depicted example of a disposable absorbent article of the present invention, the integral waistband is a region proximate to the upper perimeter of the article. In this example, the integral waistband is a gathered, elastic composite comprising elastic strands sandwiched between, and attached to, two nonwoven webs/facings. But a region of the depicted disposable absorbent article below the waistband is also an elastic composite of the same construction. A waistband need not be integral: it may be formed separately and attached to the remainder of the disposable absorbent article.

A graphic 22 comprising a colored hot-melt adhesive, or colored film or nonwoven web, is depicted as being located in the waistband on the front portion of the article, thus signifying to a user that the portion bearing the graphic is the front portion of the article. As noted elsewhere, typically instructions in the form of text, logo, symbol, or other tangible means for conveying information will be disposed on or in the package containing the article; on or in individual envelopes containing each article (to the extent one package contains a plurality of articles, and each article contained with the package is individually wrapped in, for example, an envelope or other individual container); or on or in the article itself. The instructions inform the reader of which portion of the article is signified by the graphic (e.g., the front portion of the article if the graphic is disposed on the front portion; the back portion of the article if the graphic is disposed on the back portion).

As noted elsewhere the graphic can be of many shapes including a square, a rectangle, a circle, an oval, a triangle, a line, a stripe, many other such shapes, or combinations thereof. More complex patterns and shapes may also be formed, including text and images. Also, the colored adhesive may be deposited substantially continuously or intermittently. A plurality of slot-coat modules, each having different opening shapes, or "mouthpieces," may be employed in combination to help effect complex graphics. Furthermore, if a plurality of slot-coat modules is employed, with each module at a generally fixed location with respect to a moving web, and with each module delivering a colored or non-colored adhesive to said web, then one or more modules may be operated continuously while one or more modules are operated intermittently. It should be readily understood that the deposited adhesive may be of any color, and if a plurality of adhesives are being deposited then a plurality of colors may be employed.

Accordingly, the resulting graphic or graphics may be of any color or colors. Typically, in the interests of cost, the shape and color is selected to effectively help the consumer distinguish between the front and back portion of the article, while minimizing the complexity and cost of equipment and materials used to dispose the colored hot-melt adhesive, or attach a colored film or nonwoven web, onto the disposable absorbent article or component thereof. But more sophisticated color combinations and shapes or images may be selected if justified by the value to the user of such color combinations and shapes. Furthermore, the shape and amount of the colored hot-melt adhesive may be selected to also help facilitate attachment of one component to another (or by helping facilitate attachment of one component to itself).

The depicted article in Figure 1A is representative of the many kinds of such disposable absorbent articles that may be made. For example, the front portion and back portion may correspond to regions of a single, hourglass-shaped, liquid-permeable, body-side liner overlaying, and attached to, a single, hourglass-shaped, liquid-impermeable outer cover, with an absorbent core sandwiched between these layers in the central portion of the hourglass shape. When the resulting hourglass-shaped laminate of these two layers-with the absorbent core sandwiched between these layers-is joined to itself to produce an article defining a waist opening and two leg openings, one half of the resulting article may be designated as the front portion (i.e., that portion of the disposable absorbent article that is forward of the region at which the laminate is joined to itself-e.g., to produce a seam that is proximate to a side of the hip or waist of the wearer when the article is worn), and one half of the resulting article may be designated as the back portion (i.e., that portion of the disposable absorbent article that is behind the region at which the laminate is joined to itself-e.g., to produce a seam that is proximate to a side of the hip or waist of the wearer when the article is worn). Each portion of the article so designated is generally designed to be oriented toward a specific part of the body (i.e., the front portion is designed to face the front of the body and the back portion is designed to face the back of the body).

Of course a disposable absorbent article that is completely symmetrical in all respects, such that a user could don the article with either the front portion or back portion facing the front of the user's body without any affect on performance, would likely not need a graphic signifying a specific portion of the article.

But for articles that are not symmetrical in all respects (e.g., the absorbent core's location is shifted forward or backward of a centrally located, transverse line that is parallel with the edges corresponding to the perimeter of the waist opening of the laid-flat, hourglass-shaped laminate before assembly into an article having a waist opening and two leg openings-that is more of the absorbent core is located near the front portion of the body or the back portion of the body when the corresponding article is worn; or the absorbent core's shape is, for example, generally triangular, with the base of the triangular-shaped absorbent being located in the back portion of the article; or the article employs a receptacle, opening, or other feature specifically designed to receive and help contain a bowel movement, in which case this feature will be located in the back portion of the article; or the shape of the article is such that the area or perimeter or other such physical characteristic of materials is different in the front portion compared to the back portion; etc.), then in accordance with the invention the graphic composed of a colored hot-melt adhesive, film, or nonwoven is placed to signify to the consumer that one portion of the article is different from another portion of the article (e.g., to distinguish between the front portion and the back portion).

A disposable absorbent article may be made in many other ways and include many different features. For example, the article might comprise a single, hourglass-shaped, liquid-impermeable outer cover, to which are attached other webs or components (e.g., an absorbent insert comprising a fluff/superabsorbent core encased in a liquid-permeable web could be attached to the crotch-region of the outer cover, with additional webs or panels attached to those portions of the outer cover that will be proximate to the front and back of the wearer). Or the article might be made by attaching one end of an absorbent core comprising a fluff/superabsorbent material sandwiched between a liquid-permeable, body-side liner and a liquid-impermeable outer cover to a first panel of material (e.g., a generally rectangular laminate having a liquid-impermeable outer cover attached to a liquid-permeable body-side liner), with the liquid-impermeable outer cover of the absorbent core being on the same side as the liquid-impermeable outer cover of the first panel of material. And then likewise attaching the opposing end of the same absorbent core to a second panel of similar construction to the first panel (with the liquid-impermeable outer cover of the absorbent core being on the same side as the liquid-impermeable outer cover of the second panel of material).

Many other constructions are possible. A disposable absorbent article defining a waist opening and two leg openings includes elastic materials, such as elastic strands or ribbons, in various locations, including, in accordance with the invention, in the waist region of the article; and such as near the leg openings of the article; or in various other locations to enhance fit and/or help reduce leakage. Such an article may include flaps or other such components inward of the leg openings to help produce a gasket-like fit, thereby helping to reduce leakage. Such an article may include ears or like components that are adapted to releasably engage some part of the disposable absorbent article (e.g., an ear may comprise a hook assembly that releasably engages loop or other material on the disposable absorbent article). Elastomeric films may be employed in a number of regions of the disposable absorbent article to effect an elastic quality to that portion of the article. The present invention encompasses any such construction in which a colored hot-melt adhesive, film, or nonwoven, disposed on the waistband of the disposable absorbent article, helps a user distinguish between the front portion and back portion of the disposable absorbent article.

As noted above, disposable absorbent articles generally incorporate a number of components that are assembled in a production line to produce the finished product. A colored hot-melt adhesive, film, or nonwoven may be disposed on any number of these components, so long as the resulting graphic is in the waistband portion and is visible to a user of the article.

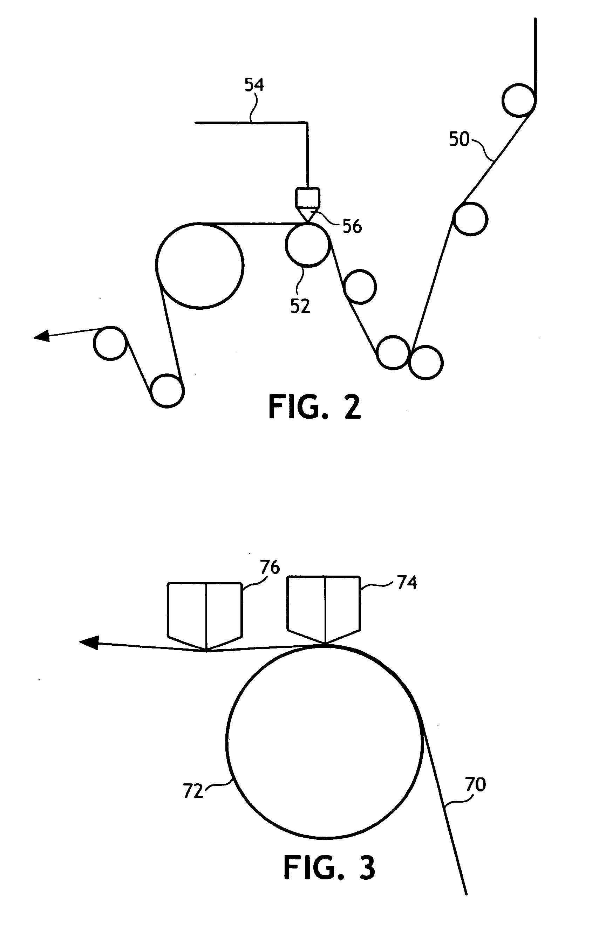

For example, in embodiments, a colored hot-melt adhesive may be disposed on the surface of a web, such as a nonwoven polypropylene web or other web, such as that is used in the assembly of a disposable absorbent article. Figure 2 depicts one representative version of a process for disposing a colored, hot-melt adhesive on the surface of a web or substrate. A web 50 is directed over a configuration of rolls to a back-up roll 52. A colored hot-melt adhesive is conducted via tubing, pipe, or other such equipment 54 to a nozzle or mouthpiece 56. As noted elsewhere, colored adhesive, likely in the form of pellets, bricks, or other solid or semi-solid form, are heated in a heating tank or other such equipment (not shown). Once liquefied such that the colored adhesive may be conveyed, it is pumped (not shown) to the nozzle or mouthpiece. Temperature, pressure, pump speed, and other such variables may be selected to give the desired flow rate and properties. A solenoid valve, or other such equipment, may be employed to provide for intermittent deposition of the colored adhesive onto the surface of the web. Furthermore, the pipe or tubing used to convey the colored adhesive, as well as the module used to dispose the colored adhesive onto the surface of a web (i.e., the module comprising a nozzle or mouthpiece used to dispose a colored adhesive onto the surface of a web or substrate), may be heated to facilitate transport and deposition of the colored adhesive.

As noted elsewhere, a plurality of slot-coating modules may be employed to effect multiple colors, graphics, and/or more complex graphics. Figure 3 depicts one representative version of a plurality of slot-coating modules being used to dispose a plurality of colored and/or non-colored adhesives onto the surface of a web. A web 70 is directed over a back-up roll 72. A first nozzle / mouthpiece 74 proximate to the surface of the back-up roll deposits a first colored or non-colored adhesive on to the surface of the web. A second nozzle / mouthpiece 76 then deposits a second colored or non-colored adhesive on to the surface of the web.

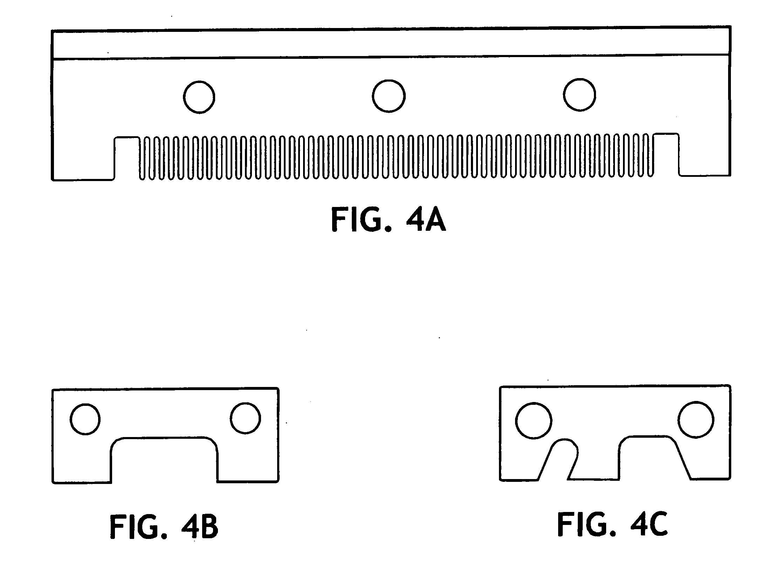

As noted elsewhere, each slot-coating module can be deployed in a variety of ways: intermittently or continuously; one color or multiple colors (i.e., a separate system of heating tanks, pipes, and pumps may be used to convey different colored adhesive formulations to the same slot-coating module or head); and different mouthpiece openings. For example, Figures 4A, 4B, and 4C give exemplary versions of mouthpiece opening shapes that might be used when depositing a colored adhesive. Figure 4A shows a comblike series of openings through which a colored adhesive or adhesives might be disposed on the surface of a web. Figure 4B shows a single opening through which a colored adhesive might be disposed on the surface of a web. And Figure 4C shows yet another representative version of mouthpiece openings through which colored adhesive might be disposed on the surface of a web, in this case through two openings, one being about twice as large as the other. Of course these figures display but a few examples of the many versions of shaped openings that might be used when disposing a colored adhesive or adhesives onto the surface of a web.

Thus, for example, a first slot-coating module employing a mouthpiece like that depicted in Figure 4A might be operated continuously to deposit a series of fine stripes along the length of the web as it travels continuously over the back-up roll. A second slot-coating module employing a mouthpiece like that depicted in Figure 4B might be operated intermittently to deposit squares or rectangles at some interval along the length of the web. These squares or rectangles might be deposited over the fine stripes. Or these squares or rectangles might be deposited adjacent to said fine stripes. It should be noted that many such combinations of slot-coating modules may be used.

As noted above, the hot-melt adhesive may be disposed on the surface of a web that is subsequently attached to another web, thereby promoting adhesive attachment between the two webs, and serving as a graphic for helping a user distinguish between the front portion and back portion of a disposable absorbent article.

As noted above, the waistband of the disposable absorbent article includes an elastic composite made of, for example, spaced-apart, substantially parallel, elastic strands or ribbons sandwiched between two nonwoven webs. Generally the elastic strand is first stretched and then attached to the webs while the strand is in a stretched condition. After the strand is attached to the webs, the resulting composite is allowed to retract, thus producing a gathered elastic composite. Thus one example of making a disposable absorbent article of the present invention would include the steps of applying a colored hot-melt adhesive on the surface of one of the webs used in making the elastic composite. A colored hot-melt adhesive, in solid form (e.g., pellets), is heated so that it is substantially liquefied. The liquefied, colored adhesive is then conveyed (e.g. by a pump or extruder or other such equipment for transporting heated liquids) to equipment (e.g., a slot coater) for disposing the adhesive onto the web. The colored hot-melt adhesive could be disposed on the web in a variety of different shapes or configurations, examples of which are given above. Note that the colored hot-melt adhesive could be disposed on the web in combination with a non-colored hot-melt adhesive (or a different color hot-melt adhesive to achieve different color combinations). For example, the colored hot-melt adhesive could be disposed on the web in the form of a discrete shape, such as a rectangle; with a non-colored adhesive disposed on other portions of the web using different equipment. Alternatively, the colored hot-melt adhesive could be disposed on the web in the form of a discrete shape; with a non-colored adhesive disposed on elastic strand (if the graphic composed of a colored hot-melt adhesive is located in or on an elastic composite comprising spaced apart elastic strands or ribbons). Many other combinations are possible, so long as the colored graphic is disposed on the waistband of the disposable absorbent article, or component thereof, in a way that the graphic is visible to a user of the article, and so that the graphic is adapted to help a user distinguish between the front portion and back portion of the disposable absorbent article. It should be noted that if a second graphic is disposed on the article, it may or may not serve to help a user distinguish between the front portion and back portion of the disposable absorbent article.

After the colored hot-melt adhesive (and perhaps additional non-colored hot-melt adhesive) have been disposed on the web, then the web can be attached to spaced-apart, substantially parallel, elastic strands and a second web to produce an elastic composite. The colored adhesive, while disposed on an interior surface of a web-that is a surface that faces and is attached to elastic strand and the second web, is visible to a user of the article comprising the colored hot-melt adhesive. This is true because webs used in the construction of disposable absorbent articles are often extremely thin. Accordingly a colored hot-melt adhesive disposed on one surface of the web is visible to an observer looking at the opposite surface on the other side of the web.

Alternatively, a colored film or nonwoven web may be used as a graphic signifying to a user the front or back portion of the disposable absorbent article. Unit operations and corresponding methods for delivering a continuous web, slitting or cutting the web, and attaching the web (e.g., a cut-and-place module or similar equipment) to another component may be used to deliver, cut, and attach a colored nonwoven web or colored film in the form of a shape such as a rectangle or square to another part of the disposable absorbent article. For example, in embodiments, a colored nonwoven web or colored film can be cut and placed between the nonwoven facings of an elastic composite comprising elastic strand sandwiched between the facings. Prior to attachment of the facings and strand to one another, the colored nonwoven web or colored film is attached, for example, to a surface of one of the facings (e.g., using an adhesive). The facing bearing the colored nonwoven web or colored film is then brought into contact with, and attached to, the elastic strands and other nonwoven facing.

Typically a seller of a disposable absorbent article of the present invention will provide instructions that communicate to a user that the graphic comprising a colored hot-melt adhesive or colored film or nonwoven identifies a specific portion of the article, thereby facilitating for the user the correct orientation of the article when donning it. So, for example, if a rectangular graphic composed of a colored hot-melt adhesive or colored film or nonwoven is centrally located in the waistband region of the back portion of a disposable absorbent article, then instructions will generally be provided that communicate to a user that the rectangular graphic is on the back portion of the article. The instructions may be in the form of text. Or the instructions may be in the form of a picture or drawing depicting the article on the human body, with that portion of the article having the rectangular graphic in facing relation to the back of the body. These instructions may be on a package, for example a plastic bag, containing a plurality of articles. Or these instructions may be on envelopes, sleeves, or other such containers enclosing individual disposable absorbent articles (if the articles are individually wrapped). Or the instructions may be on a pamphlet, card, or other such component on or in the package. Generally the instructions, whether in the form of text, logo, picture, graphic, or other such tangible means for conveying information, are made by printing on a substrate (e.g., the surface of a plastic film used in making a package that will contain a plurality of the disposable absorbent articles).

Of course the instructions reflect the location of the graphic. If the graphic was located on the front portion of the article, then the instructions would communicate to a user that the article should be donned with that portion of the article bearing the graphic in facing relation to the front of the body of the user.

The present invention also encompasses a method for helping or facilitating the donning of a disposable absorbent article. Generally the method includes the steps of positioning in the hands of a user a disposable absorbent article having a graphic composed of a colored hot-melt adhesive or colored film or nonwoven disposed thereon (whether on the front portion or the back portion)-with the graphic adapted to convey a signal to a user to help the user distinguish between the front portion and back portion of the disposable absorbent article; and then instructing (e.g., per the discussion above) the user to don the article with the graphic oriented toward the body in a way that the front portion of the article faces the front of the body of the user and the back portion of the article faces the back of the body of the user.

As noted earlier, the aforementioned inventive versions directed to marketing, packages and method for helping or facilitating the donning of a disposable absorbent article may also encompass articles comprising a first and second graphic.

### EXAMPLES

### Example 1

A nonwoven, metallocene-catalyzed polypropylene spunbond, having a basis weight of 16.96 gsm (0.5 ounces per square yard), was directed at a line speed of 393 m/min (1290 feet per minute) over a back-up roll like that depicted in Figure 2. A hot-melt adhesive, available under the designator RT2115 from Huntsman Polymer, a business having offices in Houston, Texas, was obtained in brick form. BASF Sudan Blue 670 dye was added and mixed with the adhesive at a concentration of 700 parts-per-million prior to formation of the bricks.

These bricks were placed in a heating tank and heated to a temperature of 188 degrees centigrade. The liquefied, blue-colored adhesive was then directed by a positive-displacement pump at a volumetric flow rate of 9.5 grams per minute through hoses heated to 188 degrees centigrade to a slot-coating applicator manufactured by Nordson Corporation, a business having offices in Dawsonville, Georgia. A solenoid valve was used to actuate a valve between open and closed positions so that the blue-colored adhesive passed intermittently through the applicator and associated mouthpiece. Associated with the mouthpiece was an opening having a width of 15 millimeters and a height of 0.15 millimeters. By opening and closing the valve, blue-colored rectangles were intermittently deposited on the polypropylene web. The mouthpiece was substantially perpendicular to the plane of, and contacted, the web.

The blue-colored rectangles were formed on the polypropylene web prior to the attachment of a second web, with the blue-colored rectangles sandwiched between said webs. Accordingly, contact between skin and the blue-colored adhesive was reduced or eliminated. These webs were ultimately used in the assembly of a disposable absorbent article, with the blue-colored rectangle located on the back portion of the article and proximate to the waist opening.

## Claims

1. A disposable absorbent article (10) comprising:
a front portion (16);
a back portion (18) attached to the front portion;
and a graphic (22) comprising a colored hot-melt adhesive, or a colored film or nonwoven web disposed on the disposable absorbent article, wherein the front portion of the article is attached to the back portion in a way that defines a waist opening (14) and two leg openings (12) in the disposable absorbent article; and
**characterized in that** the graphic is disposed on one of the front portion or the back portion of the article, and is adapted to convey a signal enabling a user to distinguish between the back portion and front portion of the disposable absorbent article, wherein the article comprises a waistband (20), and wherein the graphic is disposed on the article on the waistband, wherein the waistband is a gathered elastic composite comprising two nonwoven webs and one or more elastic strands or ribbons attached to, and sandwiched between the nonwoven webs, and wherein the graphic is disposed between said webs.

2. The article of claim 1 further comprising a second graphic comprising a colored hot-melt adhesive disposed on said article.

3. The article of claim 2 wherein the second graphic comprises a stripe disposed around substantially the entire circumference of the article proximate to the waist opening.

4. The article of claim 2 further comprising a plurality of stripes disposed around substantially the entire circumference of the article proximate to the waist opening.

5. A method of forming and packaging a disposable absorbent article comprising a graphic, the method comprising the steps of:
providing a nonwoven web and disposing a graphic comprising a colored hot-melt adhesive on said web, or providing a graphic comprising a colored nonwoven web or film;
assembling the web comprising the graphic, or the colored nonwoven web or film, into a disposable absorbent article having a front portion and a back portion, wherein the front portion is attached to the back portion in a way that defines a waist opening (14) and two leg openings (12) in the disposable absorbent article and wherein the article comprises a waistband (20), and wherein the graphic is disposed on the article on the waistband in one of the front or back portions of the article, wherein the waistband is a gathered elastic composite comprising two nonwoven webs, and one or more elastic strands or ribbons attached to, and sandwiched between the nonwoven webs, and wherein the graphic is disposed between said webs;
wherein the graphic is adapted to convey a signal enabling a user to distinguish between a front portion and a back portion of the disposable absorbent article; and
the method further comprises:
placing the disposable absorbent article in a package, wherein the package comprises instructions disposed on said package, and wherein the instructions can communicate to a user that the graphic can enable the user to distinguish between the back portion and front portion of the disposable absorbent article in use.

6. The method of claim 5 further comprising the step of disposing a second graphic comprising a colored hot-melt adhesive on a second nonwoven web, wherein the second nonwoven web may be the same or different from the web on which the graphic is disposed.

7. The method of claim 6 wherein the second graphic comprises a stripe disposed on the article in a location proximate to the waist opening.

8. The method of claim 7 where in the stripe is disposed substantially around the entire circumference of the waist opening.

## Patentansprüche

1. Absorptionsfähiger Einwegartikel (10), welcher umfasst:
einen vorderen Teil (16);
einen hinteren Teil (18), der an dem vorderen Teil befestigt ist;
und eine Grafik (22), die ein farbiges Schmelzhaftmittel oder eine(n) farbige(n) Film oder VI esbahn umfasst, der/die auf dem absorptionsfähigen Einwegartikel angeordnet ist, wobei der vordere Teil des Artikels an dem hinteren Teil in einer Weise befestigt ist, so dass eine Taillenöffnung (14) und zwei Beinöffnungen (12) in dem absorptionsfähigen Einwegartikel definiert sind; und
**dadurch gekennzeichnet, dass** die Grafik auf dem vorderen Teil oder dem hinteren Teil des Artikels angeordnet ist und eingerichtet ist, ein Signal zu übertragen, das es einem Benutzer ermöglicht, zwischen dem vorderen Teil und dem hinteren Teil des absorptionsfähigen Einwegartikels zu unterscheiden, wobei der Artikel einen Taillenbund (20) umfasst, und wobei die Grafik auf dem Artikel auf dem Taillenbund angeordnet ist, wobei der Taillenbund ein geraffter elastischer Verbundstoff ist, der zwei Vliesbahnen und ein oder mehrere Stränge oder Bänder umfasst, die an den Vliesbahnen befestigt und dazwischen angeordnet sind, und wobei die Grafik zwischen den Bahnen angeordnet ist.

2. Artikel gemäß Anspruch 1, welcher des Weiteren eine zweite Grafik umfasst, die ein farbiges Schmelzhaftmittel umfasst, welches auf dem Artikel angeordnet ist.

3. Artikel gemäß Anspruch 2, wobei die zweite Grafik einen Streifen umfasst, der um im Wesentlichen den gesamten Umfang des Artikels herum nahe der Taillenöffnung angeordnet ist.

4. Artikel gemäß Anspruch 2, welcher des Weiteren eine Vielzahl von Streifen umfasst, die um im Wesentlichen den gesamten Umfang des Artikels herum nahe der Taillenöffnung angeordnet sind.

5. Verfahren zum Bilden und Verpacken eines absorptionsfähigen Einwegartikels, welcher eine Grafik umfasst, wobei das Verfahren die Schritte umfasst:
Bereitstellen einer Vliesbahn und Anordnen einer Grafik umfassend ein farbiges Schmelzhaftmittel auf der Bahn, oder Bereitstellen einer Grafik umfassend eine farbige Vliesbahn oder Film;
Zusammensetzen der Bahn umfassend die Grafik oder die farbige Vliesbahn oder Film zu einem absorptionsfähigen Einwegartikel, der einen vorderen Teil und einen hinteren Teil aufweist, wobei der vordere Teil an dem hinteren Teil in einer Weise befestigt wird, so dass eine Taillenöffnung (14) und zwei Beinöffnungen in dem absorptionsfähigen Einwegartikel definiert werden, und wobei der Artikel einen Taillenbund (20) umfasst, und wobei die Grafik auf dem Artikel auf dem Taillenbund in dem vorderen oder dem hinteren Teil des Artikels angeordnet ist, wobei der Taillenbund ein geraffter elastischer Verbundstoff ist, der zwei Vliesbahnen und einen oder mehrere elastische Streifen oder Bänder umfasst, die an den Vliesbahnen befestigt sind und dazwischen angeordnet sind, und wobei die Grafik zwischen den Bahnen angeordnet ist;
wobei die Grafik eingerichtet ist, ein Signal zu übertragen, welches es einem Benutzer ermöglicht, zwischen einem vorderen Teil und einem hinteren Teil des absorptionsfähigen Einwegartikels zu unterscheiden; und
wobei das Verfahren des Weiteren umfasst:
Anordnen des absorptionsfähigen Einwegartikels in einer Packung, wobei die Packung auf der Packung angeordnete Anweisungen umfasst, und wobei die Anweisungen dem Benutzer vermitteln können, dass die Grafik es dem Benutzer ermöglichen kann, zwischen dem hinteren Teil und dem vorderen Teil des absorptionsfähigen Einwegartikels bei Verwendung zu unterscheiden.

6. Verfahren gemäß Anspruch 5, welches des Weiteren den Schritt des Anordnens einer zweiten Grafik umfasst, die ein farbiges Schmelzhaftmittel auf einer zweiten Vliesbahn umfasst, wobei die zweite Vliesbahn die gleiche sein kann oder verschieden sein kann von der Bahn, auf welcher die Grafik angeordnet ist.

7. Verfahren gemäß Anspruch 6, wobei die zweite Grafik einen Streifen umfasst, der auf dem Artikel an einem Ort in der Nähe der Taillenöffnung angeordnet ist.

8. Verfahren gemäß Anspruch 7, wobei der Streifen im Wesentlichen um den gesamten Umfang der Taillenöffnung herum angeordnet ist.

## Revendications

1. Article absorbant jetable (10) comprenant :
une partie antérieure (16) ;
une partie postérieure (18) fixée à la partie antérieure ;
et un graphique (22) comprenant un adhésif thermofusible coloré, ou bien un film ou une nappe non tissée coloré(e), disposé sur l'article absorbant jetable, la partie antérieure de l'article étant fixée à la partie postérieure de façon à former une ouverture pour la taille (14) et deux ouvertures pour les jambes (12) dans L'article absorbant jetable ; et
**caractérisé en ce que** le graphique est disposé sur l'une de la partie antérieure ou de la partie postérieure de l'article, et est adapté pour communiquer un signal permettant à un utilisateur de faire la différence entre la partie postérieure et la partie antérieure de l'article absorbant jetable, l'article comprenant une ceinture (20), et le graphique étant disposé sur l'article sur la ceinture, la ceinture étant un composite élastique froncé comprenant deux nappes non tissées et un ou plusieurs brins ou rubans élastiques fixés aux happes non tissées, et positionnés en sandwich entre celles-ci, et le graphique étant disposé entre lesdites nappes.

2. Article selon la revendication 1, comprenant en outre un deuxième graphique comprenant un adhésif thermofusible coloré disposé sur ledit article.

3. Article selon la revendication 2, dans lequel le deuxième graphique comprend une bande disposée autour de la quasi totalité de la circonférence de l'article à proximité de l'ouverture pour la taille.

4. Article selon la revendication 2, comprenant en outre une pluralité de bandes disposées autour de la quasi totalité de la circonférence de l'article à proximité de l'ouverture pour la taille.

5. Procédé de formation et d'emballage d'un article absorbant jetable comprenant un graphique, le procédé comprenant les étapes qui consistent à :
fournir une nappe non tissée et disposer un graphique comprenant un adhésif thermofusible coloré sur ladite nappe, ou fournir un graphique comprenant une nappe non tissée ou un film colore(e) ;
assembler la nappe comprenant le graphique, ou bien a nappe non tissée ou le film coloré(e), en l'intégrant dans un article absorbant jetable comportant une partie antérieure et une partie postérieure, la partie antérieure étant fixée à la partie postérieure de façon à former une ouverture pour la taille (14) et deux ouvertures pour les jambes (12) dans l'article absorbant jetable et l'article comprenant une ceinture (20), et le graphique étant disposé sur l'article sur la ceinture dans l'une de la partie antérieure ou de la partie postérieure de l'article, la ceinture étant un composite élastique froncé comprenant deux nappes non tissées et un ou plusieurs brins ou rubans élastiques fixés aux nappes non tissées, et positionnés en sandwich entre celles-ci, et le graphique étant disposé entre lesdites nappes ; le graphique étant adapté pour communiquer un signal permettant à un utilisateur de faire la différence entre une partie antérieure et une partie postérieure de l'article absorbant jetable ; et
le procédé comprenant en outre :
le fait de placer l'article absorbant jetable dans un emballage, l'emballage comprenant des instructions disposées sur ledit emballage, et les instructions pouvant indiquer à un utilisateur que le graphique peut permettre à l'utilisateur de faire la différence entre la partie postérieure et la partie antérieure de l'article absorbant jetable en cours d'utilisation.

6. Procédé selon la revendication 5, comprenant en outre l'étape qui consiste à disposer un deuxième graphique comprenant un adhésif thermofusible colore sur une deuxième nappe non tissée, la deuxième nappe non tissée pouvant être identique à la nappe sur laquelle le graphique est disposé ou différente de celle-ci.

7. procédé selon la revendication 6, dans lequel le deuxième graphique comprend une bande disposée sur l'article à un emplacement situé à proximité de l'ouverture pour la taille.

8. Procédé selon la revendication 7, dans lequel la bande est disposée autour de la quasi totalité de a circonférence de l'ouverture pour la taille.
